# EUROPEAN PATENT SPECIFICATION

(11) **EP 0 358 692 B1**
(45) Date of publication and mention of the grant of the patent: **03.06.1998**
(21) Application number: 88904351.9
(22) Date of filing: 29.04.1988
(51) Int. Cl.: C07K 2/00, C07K 14/00, C07H 15/00, A61K 39/00, A61K 41/00

(54) **CHOLERA VACCINES**
IMPFSTOFF GEGEN CHOLERA
VACCINS CONTRE LE CHOLERA

(30) Priority: 29.04.1987 US 43907
(43) Date of publication of application: 21.03.1990
(73) Proprietor: PRESIDENT AND FELLOWS OF HARVARD COLLEGE, Cambridge, MA 02138 (US)
(72) Inventor: MEKALANOS, John, J., Cambridge, MA 02138 (US); TAYLOR, Ronald, K., Memphis, TN 38163 (US)
(74) Representative: Moon, Donald Keith
(86) International application number: US8801409
(87) International publication number: WO8808431

(56) References cited:
- Infection and Immunity, Vol. 53, No. 2, issued August 1986, "Molecular Cloning and Expression in Escherichia Coli K-12 of the O Antigens of the Inaba and Ogawa Serotypes of the Vibrio Cholerae 01 Lipopolysaccharides and Their Potential for Vaccine Development" pages 272-277, (MANNING)see Summary. (Australia)
- FEBS, Vol. 208, No. 2, issued November 1986, "Immunoactive Chimeric ST-LT Enterotoxins of Escherichia Coli Generated by in Vitro Gene Fusion" pages 194- 198(SANCHEZ)see Summary. (Sweden)
- Infection and Immunity, issued May 1986, Vol. 52, No. 2, "Immunity to Heat- Labile Enterotoxins of Porcine and Human Escherichia Coli Strains Achieved with Synthetic Cholera Toxin Peptides" pages 562-567(JACOB)see Summary. (Missouri)
- Infection and Immunity issued May 1978, pages 552-558, Vol. 20, No. 2, (MEKALANOS) "Purification of Cholera Toxin and its Subunits: New Methods of Preparation and the use of Hypertoxinogenic Mutants" (see Summary). (Los Angeles, California).
- Biological Abstract, Vol. 73, issued 1982, (KANOH) "Transferable R Plasmids Isolated from Vibrio Cholerae Eltor" Abstract No. 31709 (Philadelphia, Pa).
- Advances in Research on Cholera and Related Diarrheas, 3, eds., S. KUWAHARA, N. F. PIERCE, pages 271-275, issued 1986, (Black) "Oral Immunization with Killed Whole Vibrio and B Subunit or Procholeragenoid Combination Cholera Vaccines: Immune Response and Protection from V. Cholerae Challenge" (Switzerland) see paragraph 1.
- Infection and Immunity, issued November 1984, pages 564-569, Vol. 46, (CLEMENTS) "Construction of a Potential Live Oral Bivalent Vaccine for TyphoidFever and Cholera-Excherichia Coli- Related Diarrheas" (Louisiana) see Summary.
- Vaccines, Vol. 85, issued 1986, pages 107-111 (KAPER) "Development and Testing of a Recombinant Live Oral Cholera Vaccine" (Baltimore MD.) see Summary.
- Infection and Immunity, issued November 1984 pages 465-469, Vol. 46, No. 2, (FORMAL) "Oral Vaccination of Monkeys with an Invasive Escherichia Coli K-12 Hybrid Expressing Shigella Flexneri 2a Somatic Antigen" (Bethesda, MD.) see Summary.
- Infection and Immunity, issued May 1984, pages 268-273, Vol. 44, No. 2, (KLIPSTEIN) "Properties of Cross-Linked Toxoid Vaccines Made with Hyperantigenic Forms of Synthetic Escherichia Coli Heat-Stable" see Summary. (California)
- Microbiological Reviews, issued June 1982, pages 129-161, Vol. 46, No. 2, (GAASTRA) "Host-Specific Fimbrial Adhesins of Non-Invasive Enterotoxigenic Escherichia Coli Strains" see Introduction.
- Acute Enteric Infections in Children, issued 1981, Chapter 26, pages 443-459, (LEVINE)"Volunteer Studies in Development of Vassines against Cholera and Enterotocigenic Escherichia Coli:A Review" (See Introduction) (Columbia, Mo.)
- CHEMICAL ABSTRACT, Vol. 101, issued 1984, (MEL'NIKOVA) "Study of Natural Plasmids P and Vib of the Choler Vibrio" (Columbus, OH.) Abstract No. 104975s.
- CHEMICAL ABSTRACT, Vol. 107, issued July 1987, Abstract No. 1732e, (TAYLOR) "Use of PhoA Gene Fusions to Identify a Piluscolonization Factor Coordinaterly Regulated with Cholera Toxin" (Columbus OH.)
- Infection and Immunity, issued June 1977, pages 789-795, Vol. 16, No. 3, (Los Angeles California) "Simple Method for Purifying Choleragenoid, the Natural Toxoid of Vibrio Cholerae" (MEKALANOS) see Summary.
- N. Harford et al., Nature, 306(8), 1983, 551-557
- Evidence filed with letter dated 24.06.93
- Affidavits by Dr. Mekalanos and Dr. Taylor filed with letter dated 04.11.93
- Affidavit by Dr. Mekalanos filed with letter dated 15.03.96

## Description

### Background of the Invention

This application is a continuation-in-part of Mekalanos, entitled "Cholera Vaccines", United States Serial No. 043,907, assigned to the same assignee as the present application and the whole specification and figure of which is hereby incorporated by reference.

This invention relates to immunological protection against Vibrio cholerae.

V. cholerae is a bacterial species that can cause a diarrheal disease in humans by colonizing in the small intestine and secreting a protein toxin. The action of the cholera toxin has been well characterized. The toxin includes two subunits, the A and B subunits; the B subunit has no known toxic activity but provides a degree of immunological protection when used as a component of a vaccine. Black et al. 1986 in Advances in Research on Cholera and Related Diarrheas 3:271. eds. Kuwahara et al.

In addition to B sub-unit vaccines, vaccines for cholera also include denatured whole cholera toxin, killed whole cells of V. cholerae grown usually on a solid medium at pH 7.5-8.0, and mixtures of killed cells and inactivated toxin molecules. Other vaccines include live attenuated V. cholerae strains which do not produce the A subunit of cholera toxin, and attenuated strains of heterologous non-Vibrio cholerae carriers, that is, non-V. cholerae strains, e.g., Salmonella typhi Ty21A having cloned genes encoding the cholera protective 01 antigen (J. Infectious Desease 131:553-558, 1975).

Ehara et al. (Trop. Med. 28:21, 1986; and Vaccine, 1988) describe purification of fimbriae of V. cholerae having a structural subunit protein of about 16 kD. This protein is not stained by Coomassie blue and its haemagglutination (HA) titre is mannose sensitive. AL-Kaissi et al. (J. App. Bact. 58:221, 1985) also describe fimbriae which have a mannose sensitive HA titre.

Kanoh (Nippon Saikingaku Zasshi, 36:465, 1981), Melnikova et al. (Mol. Biol. Genet. Russian 2:18, 1982), and Kanoh (Jpn. J Bacteriol. 36:465, 1981) describe sex pili of V. cholerae, encoded by plasmid genes. Holmgren et al. (Infect. Immun. 33:136, 1981) describe various E. coli fimbriae.

Taylor et al, Bacterial Vaccine and Local Immunity, (Ann Sclavo 1986 n. 1-2, 51-61), reported that a number of colonization deficient V. cholerae mutant strains were found to lack a 20.5 Kd protein which was identified as a pilus protein and was found to be coordinately regulated with cholera toxin.

Shaw et al. American Society for Microbiology, Abstracts of the Annual Meeting held Atlanta, USA, 1 - 6 March 1987, reported that analysis of the cloned TcpA gene of V. cholerae showed that the pili protein had similarity to other pili proteins including a modified N-terminal amino acid, a hydrophobic domain and a potential cysteine loop.

### Summary of the Invention

It has been found that a V. cholerae surface protein structure called a pilus, specifically a toxin-coregulated pilus ("TcpA pilus") is instrumental for adherance of V. cholerae to the intestine and for colonization in the intestine. Production of this pilus is significantly enhanced under certain laboratory culture conditions. The B subunit of cholera toxin, is co-regulated with production of TcpA pilus and is also useful as a vaccine component. In addition, we have cloned the structural gene for the TcpA pilus subunit and determined the inferred amino acid sequence of the TcpA protein. The use of these materials and information enables production of several different types of cholera vaccines that are able to stimulate production of protective antibodies that recognize the TcpA pilus and thus inhibit adherance to, and colonization of, the human intestine by V. cholerae.

Accordingly, in a first aspect the invention provides a method for producing a cholera vaccine comprising growing a culture of V. cholerae cells having a mutated htx gene in growth medium wherein the toxin-coregulated pilus is present at a concentration representing 1% or more of total cell protein and inoculating an animal with cells or cell components from said culture.

In a second aspect the invention features a method for producing a cholera vaccine comprising growing a culture of V. cholerae cells in growth medium at a pH of about 6.5 or less wherein the toxin-coregulated pilus is present at a concentration representing 1% or more of total cell protein and inoculating an animal with cells or cell components from said culture.

Preferably, the vaccine is a whole cell vaccine and the method further comprises killing the cells; or the vaccine comprises a TcpA pilus, and the method further comprises harvesting the V. cholerae and purifying the TcpA pilus from the cells; or the vaccine comprises B subunit of cholera toxin and the method further comprises harvesting the V. cholerae cells and purifying the B subunit of cholera toxin from the cells.

The invention also features a bacterial cell having at least 1% total protein as TcpA. Preferably the cell lacks toxic levels of cholera toxin; and most preferable the cells is a Vibrio cholerae having a mutated htx gene.

### Description of the Preferred Embodiments

### Structure

### TcpA pilus

The Figure shows the DNA sequence, and corresponding amino acid sequence, of the gene encoding TcpA pilus in V. cholerae.

The TcpA pilus is an example of a filamentous surface component (pilus) of V. cholerae cells. When isolated from whole cells of V. cholerae 0395 it has an apparent molecular weight in SDS polyacryamide gel electrophoresis of 20.5KD. The TcpA pilus is associated with the macromolecular filamentous structures on the surface of V. cholerae and can be partially purified by differential centrifugation. The pilus is produced in significant quantities only under certain environmental conditions of growth of V. cholerae cells. For example, its production is best at a low pH, preferably pH 6.5 or less, and is virtually undetectable at a pH above 7.5.

Further, its production is best at a low ionic strength equivalent to between 50-100 mM NaCl. Other factors controlling TcpA pilus production include the presence of amino acids in the growth medium (e.g. LB, see below), aeration (e.g., 0.5-2 liter/min per 6 liter of medium), the type of medium (preferably liquid), and incubation temperature (preferably 25-30°C). In addition, the TcpA pilus is only produced at a particular stage of growth of the V. cholerae cells (e.g., stationary phase). These growth conditions generally are not optimal for cellular growth; rather they are optimal for production of TcpA pilus.

TcpA pili form bundles of filaments when purified, the protein is stained by Coomassie blue, and its haemagglutination activity is not affected by mannose.

To measure the percentage of TcpA in a preparation, a portion of the preparation is run in a gel, reacted with antibody to TcpA to locate the protein band corresponding to TcpA, the band is cut from a gel, and the amount of TcpA is determined. Total protein is determined by standard procedure, and used to calculate % TcpA.

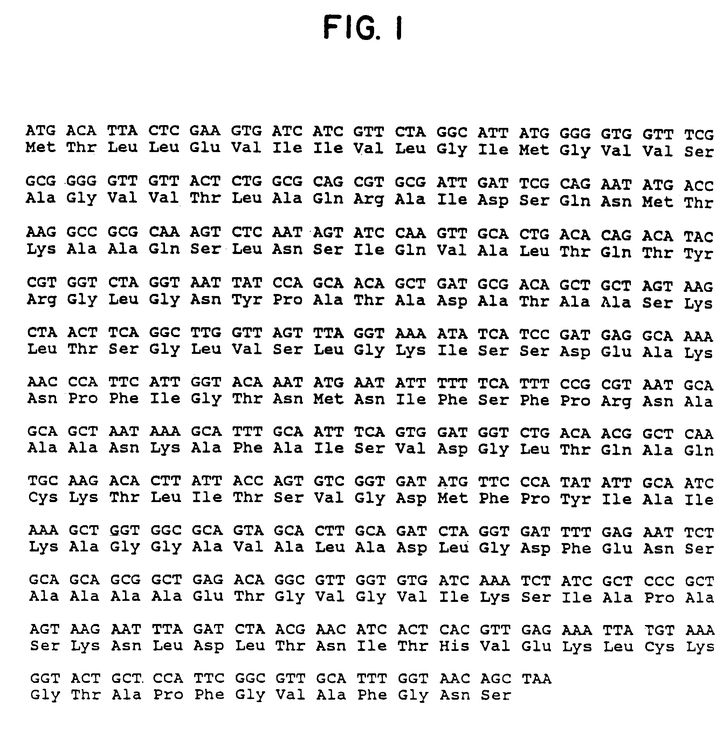

The gene encoding TcpA pilus in V. cholerae has been cloned, as described below; its DNA sequence is shown in Fig. 1. The TcpA gene is chromosomally located. Uusing this cloned DNA, related DNA from other strains of V. cholerae, or synthetic DNA encoding substantially the same amino acid sequence shown in the figure, the TcpA pilus can be produced in other bacterial strains, using standard procedures. Thus, the term TcpA pilus includes proteins having amino acid modifications which do not substantially affect the antigenicity of the molecule. Similarly the gene encoding TcpA includes genes encoding for such amino acid modifications, or for antigenic portions of TcpA. For example, the DNA can be introduced into any standard vector and transformed into bacterial strains capable of expressing the TcpA pilus; an example of this is given below.

### Example 1: Cloning the TcpA gene

TnphoA is a derivative of the transposon Tn5 that retains its broad host range and random insertion properties but in addition can create fusions between target genes and phoA, the alkaline phosphatase gene of Escherichia coli (Manoil et al. 82 Proc. Nat'1 Acad. Sci USA 8129, 1986). Such gene fusions encode hybrid proteins composed of a carboxy terminal portion of alkaline phosphatase (PhoA) fused in frame to an amino terminal portion of a target gene product. These hybrid proteins exhibit little or no PhoA activity unless the target gene encodes a secreted or membrane spanning protein. These active fusions are easily identified as blue colonies by incorporating the chromogenic alkaline phosphatase substrate 5-bromo-4-chloro-3-indolyl phosphate (XP) into agar medium on which the bacterial cells carrying inserts of TnphoA have been plated. Because virtually all bacterial proteins implicated as virulence factors are periplasmic, extracellular, or cell surface associated, this method provides a strong enrichment for insertion mutations that may affect pathogenic properties of bacteria, such as colonization or toxin production.

Random insertions of TnphoA into the chromosome of V. cholerae was accomplished through the use of pRT291 a derivative of the broad host range P-group plasmid pRK290 (Mekalanos, Nature 276:633, 1978) that carries a copy of TnphoA (constructed by spontaneous transposition with TnphoA). Briefly, chromosomal inserts of TnphoA were obtained in V. cholerae strains carrying pRT291 by superinfection with the incompatible plasmid pPhlJI (Id.) and selection for resistance to kanamycin and gentamycin. V. cholerae colonies carrying inserts of TnphoA were screened for the PhoA⁺ phenotype on LB agar containing 0.2% glucose and 20 mg/ml of XP at 30°C.

A pool of several thousand V. cholerae colonies carrying inserts of TnphoA was screened for mutations that encoded PhoA⁺ fusion proteins. About 1% of the colonies carrying inserts of TnphoA were blue on this medium indicating that they carry copies of TnphoA fused to genes encoding secreted or membrane proteins that express in this medium.

Forty of these blue colonies were purified and analyzed for their total protein profile by polyacrylamide gel electrophoresis (PAGE). V. cholerae wild-type and mutant strains were grown in LB broth, pH 6.5 at 30°C for 18 h with aeration. Cells were collected by centrifugation, lysed in sample buffer with a reducing agent, and analyzed by electrophoresis through a 12.5% polyacryamide slab gel in the presence of NaDodSO₄, as previously described by Mekalanos et al. (16 Infect.Immun. 787, 1977).

Seven mutants have detectable differences in their protein profile showing the loss of one or more bands. One type of mutant, represented by strain RT110.21, has lost a single protein of molecular weight 20.5 KD.

A mutant lacking the 20.5 KD protein was tested for colonization defects by a competition assay involving coinfection of the mutant with the parental strain into 3-9 day old CD-1 mice essentially as described by Freter et al. 34 Infect. Inmun. 234, 1981. The mutant strain RT110.21, which had lost the 20.5 KD protein, showed a marked decrease in its ability to compete with the parental strain in vivo, but not in vitro. Other competition experiments performed with another independently isolated mutant lacking the 20.5 KD protein confirmed that this class of mutant is severely defective in colonization in both infant mice as well as adult rabbits.

Cell fractionation showed that the 20.5 KD protein is associated with macromolecular structures that are located on the bacterial cell surface. A nonflagellated mutant (mot-39; Mekalanos, 306 Nature 551, 1983) derived from strain 0395-N1 was used to partially purify these structures from sheared cells by several rounds of differential sedimentation. The strain was grown in LB broth, pH 6.5. A volume of 400 ml of broth per 2 liter flask was used and the culture was incubated at 150 revolutions per min for 16 h at 30°C. The cells were collected by centrifugation and resuspended in a buffer containing 12.5 mM tris-HCl pH 7.0, 25 mM NaCl, 4mM MgCl₂, and 4 mM CaCl₂. After shearing the bacterial cells by passage through a 21 guage needle, the pili were purified by several rounds of differential centrifugation to remove cells and soluble proteins. Typically, sheared cells are centrifuged at 3000 X G for 20 minutes. Supernatant fluid is collected and the pellet discarded. The supernatant fluid is than centrifuged at 15,000 X G for 40 minutes. The supernatant from this step is discarded and the pellet collected and resuspended in (TCP buffer). These two steps of differential centrifugation are repeated twice again. The final pellet represents purified TcpA (i.e., TcpA protein represents greater than 50% of the total cell protein) in such as preparation. The isolation of the pili was monitored by following the purification of the 20.5 kilodalton pilus subunit by PAGE. Purified pili were observed under the electron microscope after staining preparations with 2% ammonium molybdate. Examination of these preparations in the electron microscope showed long laterally associated fimbria or pili (7nm in diameter). Individual pilus filaments could be seen on the surface of cells of strain 0395 but were not seen on cells of TnphoA induced mutants that had lost the 20.5 KD protein.

The 20.5 KD protein was further purified by electroelution after PAGE and subjected to N-terminal amino acid sequence analysis. The sequence data corresponds to that shown in the Figure. This sequence is highly hydrophobic and may represent part or all of a secretory signal sequence. Consistent with this conclusion, subcloning of the TnphoA fusion from strain RT110.21 and DNA sequencing has shown that the phoA gene is fused to the pilus coding sequence 92 codons downstream from the sequence encoding this hydrophobic stretch of amino acids. Two other mutants which have lost the 20.5 KD protein also have TnphoA inserts that have fused phoA to this same open reading frame, confirming that this sequence does indeed represent the structural gene for the V. cholerae pilus.

We have cloned the gene for the tcpA gene in two steps. First, the tcpA-TnphoA gene fusion carried by RT110.21 was cloned onto a plasmid by selecting for its kanamycin resistant phenotype in E. coli. A gene probe derived from the DNA sequences adjacent to this TnphoA fusion was then used to identify a cosmid clone carrying the wild type tcpA gene of strain 0395 (Id.). This plasmid, called pCS12G7, carries an active tcpA gene as shown by the following experiments:
a. When pCS12G7 is introduced into the tcpA mutant strain RT110.21, it complements its pilus defect and the plasmid carrying strain once again produces the TcpA pilus.
b. DNA sequencing of the entire tcpA gene confirmed its location on pCS12G7. This sequence is presented in the figure.
c. When introduced into V. cholerae strains 0395-N1 or 569B-N1, pCS12G7 induces a hyper-piliation phenotype in which these strains produce 2-3 times more TcpA pili than usually produced in expression media. 569B-N1 is an Inaba serotype strain of V. cholerae. It carries a deletion in the ctxA gene but is ctxB+. The strain was constructed by transduction of the ctxAB Km^{r} mutation of 0395-NT into a spontaneously highly motile derivative of 569B (strain 569B is usually non-motile). The transductant (strain 569B-NT) was then converted to 569B-N1 by recombination with pJM290.2 (Id.).
d. When introduced into E. coli or Salmonella typhimuruim LB5000, pCS12G7 produces a protein subunit that is identical in size and immunological properties to the TcpA protein by Western blot analysis.

### B subunit of cholera toxin

The B subunit is present in cholera toxin at a ratio of 5:1 with A subunit. It has no known toxic activity and is useful as a component for vaccines against cholera. Strains of V. cholerae having mutations which inactivate the A subunit are known. The level of both A and B subunits can be markedly increased by growing V. cholerae cells in medium suitable for production of the TcpA pilus, as described above. By using mutants having inactive A subunit e.g. ctxA deletion strains (Id.), it is possible to grow cells to produce large amounts of both the B subunit and TcpA pilus simultaneously.

The reason for enhanced production of B subunit and TcpA pilus appears to be that the genes encoding these proteins are regulated by the same gene--toxR--the regulation or activity of which in turn is affected by growth conditions.

Furthermore, by using a tcpA-specific hybridization probe (a DNA fragment containing only nucleotides known to encode amino acids of the TcpA protein), we have shown that the tcpA gene is present in all strains of V. cholerae isolated from patients with cholera. In contrast, several environmental strains of V. cholerae (i.e., strains isolated from water sources, rather than patients), do not contain the tcpA gene. These observations support the contention that tcpA encodes the colonization factor for all V. cholerae strains implicated in human diarrheal disease. In addition, environmental strains of V. cholerae which do not contain the tcpA gene are known to colonize human volunteers poorly and induce little if any immunity in those volunteers. Levine et al., In Acute Enteric Infections in Children, Ch. 26., 1981 Elsevier. The explantation for this is that those strains cannot make the TcpA pilus and therefore cannot colonize humans or induce protective antibodies that react with the TcpA pilus.

### Cholera Vaccines

The ability to produce both TcpA pilus and large amounts of B subunit make possible the construction of a variety of cholera vaccines.
a. Killed whole cell vaccine

For this vaccine V. cholerae ceils are grown under conditions that allow high expression of the TcpA pilus and/or the B subunit of cholera toxin. We have established such conditions for strains 0395-NI, 569B-NI, and their derivatives (see Example 2 below) but have shown that these conditions can be similarly established for theoretically any strain of V. cholerae provided TcpA and cholera toxin B subunit production are used to optimize growth parameters. Similarly, mutant strains of V. cholerae can be isolated that produce TcpA and B subunit under growth conditions different from those presented here (see strain 569B htx-5 in example 2 below). The essential aspect of our invention is that cultivation of V. cholerae under certain growth conditions can produce cells that express TcpA pili on their surface at high level (i.e., greater than 1% of total cell protein) and thus form a suitable cholera vaccine. Once appropriate strains are grown under appropriate conditions, the cells are then killed by standard procedures, such as by treatment with glutaraldehyde or formalin. These treatments must be such as to preserve the antigenic properties of the TcpA. pilus and B subunit.

This vaccine can be supplemented with purified B subunit produced by standard procedure, or by growing cells under conditions which enhance its production, and then purifying the protein by standard procedure.

### Example 2:

This example demonstrates the production of cholera B subunit and piliated V. cholerae cells for use in killed whole cell vaccine or for preparation of purified TcpA pilus.

At least three different strains of V. cholerae can be used for production of piliated ceils and the 3 subunit of cholera toxin. Strains 0395-N1 and its hyperpiliated derivative 0395-N1 (pCS12G7) are Ogawa in serotype while strains 569B-N1 and its hyperpiliated, hypertoxigenic derivative 569B-N1 htx-5 are Inaba in serotype. Strain 569B-N1 htx-5 is a derivative of strain 569B-N1 carrying a htx mutation isolated by standard methods (Mekalanos et. al Proc. Natl. Acad. Sci. USA 75:941 1978. We have found that in addition to causing hyperproduction of cholera toxin, the htx mutation also causes hyperproduction of the TcpA pilus under both the growth conditions outlined below as well as other growth conditions that are not usually permissive for TcpA production (e.g., high pH's such as 7.5). A cholera killed whole cell vaccine should preferably contain cells of both the Ogawa and Inaba serotypes and therefore separate cultures of one Ogawa and one Inaba strain are normally prepared. The hyperpiliated derivatives produce 2-3 times as much TcpA pilus as their parental derivatives because they either carry the high copy number plasmid pCS12G7 that contains the tcpA gene of strain 0395 (i.e., 0395-N1 pC512G7) or they carry the htx mutation (i.e., 569B-N1 htx-5). All strains are cultured as described below except that ampicillin is added to the medium to a final concentration of 50 micrograms per ml for strain 0395-N1 (pCS12G7).

Starter cultures of the selected V. cholerae strain are prepared in test cubes containing 2 ml of LB-6.5 medium (10 g Tryptone, 5 g yeast extract, 5 g sodium chloride, pH adjusted to 6.5 before autoclaving) and incubated at 30° C on a roller incubator at a speed of 30 RPM of 18 h. Starting cell density of the starter culture is low (about 10⁷ per ml inoculated from a fresh agar plate culture) and after growth, autoagglutination of the bacterial cells is apparent as clumps of material easily visible to the naked eye. The bacterial cells that are clumped are allowed to settle out of the upright stationary tube and then collected from the bottom with a pipet. The clumped cells are used to inoculate 6 liters of LB-6.5 contained an appropriately designed fermentation vessel that allows air to be pumped through the medium and dispersed as fine bubbles. The culture is incubated with moderate aeration (0.5-1 liter/min) at 25° C for 24 h during which time the culture reaches stationary phase and becomes nearly completely autoagglutinated. The clumped bacterial cells carrying TcpA pili on their surface are collected by centrifugation and resuspended in 600 ml of 0.85% sodium chloride, 10mM sodium phosphate buffer pH 7.0.

These piliated V. cholerae cells are then used for preparation of purified TcpA pili or killed whole cells while the cell free culture supernatant fluid is used to purify the B subunit of cholera toxin by standard methods (Mekalanos et al., Infection and Immunity 16:789, 1977 and 20:552, 1978).

It is anticipated that the method used to kill and preserve these piliated cells for use in the whole cell vaccine should not destroy the immunogenicity of the TcpA pili. In this regard, treatment of the piliated cells for 24-48 h with 1% glutaraldehyde or 1% formalin effectively kills the organism while maintaining the immunogenicity of the TcpA pilus. The formulation of one oral or parenteral dose of this killed whole cell vaccine should preferably include at least 10¹⁰ killed piliated V. cholerae cells of the Ogawa serotype (0395-N1 or 0395-N1 (pCS12G7)) and at least 10¹⁰ killed piliated V. cholerae cells of the Inaba serotype (5693-N1 or 569B-N1 htx-5).

The protective efficacy of such a vaccine can be further improved by including 200-500 micrograms of the purified B subunit of cholera toxin prepared from the same cultures used to prepare the piliated V. cholerae cells. Note that all four production strains are deleted for the A subunit of the toxin and that the above culture conditions are optimal for expression of both the TcpA pilus and the B subunit--these are the preferred types of production strains and conditions.

Alternatively, the piliated cells above could be sheared and the TcpA pilus purified by differential sedimentation, or other methods, and then used either alone or combined with the purified cholera B subunit as a vaccine.

### b. TcpA pilus vaccine

This vaccine is composed of at least an immunogenic fragment of TcpA pilus (e.g., a fragment having at least one TcpA pilus determinant and preferably at least two or three such determinants). The pilus can be prepared either synthetically or from whole cells grown under appropriate conditions and then purified by standard procedure, or it can be prepared by using recombinant DNA methology. For example, a synthetic polypeptide corresponding to a fragment of the TcpA pilus amino acid sequence can be prepared by standard procedures, or a nucleic acid sequence encoding such a fragment may be expressed in an expression system, and the resulting polypeptide purified. Standard procedures can be used to identify such fragments, for example, partial deletions of the tcpA gene can be constructed, expressed as described above, and the efficacy of this partial TcpA product studied. Those fragments that have immunogenicity similar to the native TcpA pilus would be useful in such a vaccine. Similarly, polypeptides which are cross-reactive with the TcpA pilus are suitable in this invention. (By cross-reactive is meant those polypeptides which are immunoprecipitable with antibodies produced to TcpA pilus.) See generally, J. Mol. Immunol. 19:1541-1549, 1982; and J. Mol. Immunol. 21:785-793, 1984.

This vaccine may be supplemented with killed whole cells of V. cholerae, with B subunit, or may be used to supplement existing vaccines.

Synthetic peptides also offer an inexpensive means for producing a pure immunogen for use in vaccines. The deduced amino acid sequence of the TcpA pilin (derived from its DNA sequence) provides the essential information needed to design a synthetic peptide that might serve as an immunogen for raising antibodies that react with the TcpA pilus and block its function (cell binding). Such immunogenic peptides can be identified by a systematic approach in which non-overlapping or partially overlapping peptides are synthesized and then antibodies are raised to each. The peptides that induce antibodies which either react with and inhibit binding of the TcpA pili to host cells, or actually protect animals from virulent V. cholerae are then identified. These peptides carry the protective epitopes of the TcpA pilus and can therefore be used as a cholera vaccine, preferably after chemically crosslinking them to an appropriate immunologic carrier protein. The carrier protein enhances the immunogenicity of the peptide by providing "T-cell help functions." Many different proteins have been used as peptide carriers but one of the best is cholera B subunit or the B subunit of the heat-labile enterotoxin of E. coli (Infection & Immunity 44:268-273, 1984). Example 3: TcpA-related Chimeric Protein Vaccine

In the same way that a chemically crosslinked TcpA-related peptide carrier protein conjugate can be used as an immunogen, genetically derived fusion proteins can also serve as immunogens in a cholera vaccine. These gene fusions are made from the structural gene for the TcpA pilus or from a synthetic DNA oligonucleotide that encodes peptide sequences related to the TcpA protein, and the gene for the carrier protein. We have made such a gene fusion between the tcpA gene and the gene for alkaline phosphatase (phoA) of E. coli and, as described above, have demonstrated the production of a fusion protein in both V. cholerae and E. coli. These fusion proteins react with antibody raised against TcpA pili and therefore would probably stimulate antibodies against the TcpA pilus if the fusion proteins were used as immunogens. This genetic approach can also be utilized to make other fusion proteins between tcpA-related DNA sequences and genes for carrier proteins like LT-B subunit cholera toxin B subunit, diptheria toxin, and tetanus toxin (FEBS Letters 208:194-198 (1986)).

### c. Heterlogous live vaccines

Living cells of Salmonella, E. coli or vaccinia virus, which have been modified to be relatively non-pathogenic are transformed, or otherwise modified by recombinant DNA methodology, to encode TcpA pilus protein and preferably also the B subunit of cholera toxin. These cells or particles may be used to inoculate against cholera if they can stimulate antibody production against TcpA pilus and preferably also 3 subunit. Only an immunologically active fragment of either protein need be encoded by these organisms, and this vaccine can be used in conjunction with the above vaccines, or with prior vaccines.

### Example 4:

This example describes the construction of a live heterologous carrier vaccine expressing the TcpA pilus subunit, Several organisms including S. typhi Ty21A (J. Infect. Dis. 131:553, 1975; Inf. & Immunity 46:564-569, 1984), other Salmonella species (Nature 291:238-239, 1981), E. coli-Shigella hybrids (Inf. & Immunity 46:465-469, 1984), and vaccinia virus (Nature 311:67, 1984; Proc. Natl. Acad. Sci. USA 80:7155, 1983), are potential live carrier vaccines that will not only immunize against homologous diseases (typhoid fever, shigellosis, small pox, etc.) but also against other diseases carried by the live carrier strain and expressed by genes for the appropriate protective immunogens. Both E. coli and S. typhimurium strains carrying the plasmid pCS12G7 or related plasmids, such as pCS12G10, produce a protein that is immunologically identical to the TcpA pilin. Thus, this plasmid or derivatives of this plasmid will provide the necessary genetic information for live carrier vaccine strains to express TcpA-related immunogens.

The method for preparation of this vaccine depends on the organism used as the live carrier vaccine strain. For bacterial carriers, plasmids expressing the TcpA pilin are introduced by transformation or conjugation, using standard procedure. Such plasmids can be integrated into the chromosome of the carrier strain to provide more stable genetic inheritance of the TcpA pilin gene. For viral carriers, the tcpA gene sequence is genetically engineered to express in host (animal or human) cells that are infected with the recombinant virus.

The efficacy of a live heterologous carrier vaccine expressing the TcpA pilin will be improved if it also expresses the B subunit of cholera toxin. The introduction of the ctxB gene as well as the tcpA gene into the same strain of carrier organism is therefore preferred.

Once constructed, a live heterologous vaccine strain is compounded into a vaccine by standard procedures and administered orally or parenterally in a dose that is large enough to allow the organisms to multiply, but not cause overt disease in the vaccinated host. (e.g. about 10⁶-10¹⁰ cells or virus particles per dose)

### Use

The use of the above vaccines includes their inoculation into humans or other animals to prevent cholera and related infections. The vaccines can be administered using standard procedure, preferably by oral route, but also by injection. Dosages will vary from about 10⁹-10¹⁰ live bacteria or 10¹⁰ killed bacteria, and from 10-1000mg TcpA pilus or B subunit per kg animal body weight.

### Deposits

The following deposits were made on April 29, 1987, with the American Type Culture Collection (ATCC), where the deposits were given the following accession numbers:

| Deposit | Accession No. |
|---|---|
| V. cholerae 0395-N1 (pCS12G7) | 67396 |
| V. cholerae 569B-N1 htx-5 | 53613 |
| S. typhimurium LB5000 (pCS12G10) | 67397 |

Applicants' assignee, President and fellows of Harvard College, represents that the ATCC is a depository affording permanence of the deposit and ready accessibility thereto by the public if a patent is granted. All restrictions on the availability to the public of the material so deposited will be irrevocably removed upon the granting of a patent. The material will be available during the pendency of the patent application to one determined by the Commissioner to be entitled thereto under 37 CFR 1.14 and 35 USC 122. The deposited material will be maintained with all the care necessary to keep it viable and uncontaminated for a period of at least five years after the most recent request for the furnishing of a sample of the deposited microorganism, and in any case, for a period of at least thirty (30) years after the date of deposit or for the enforceable life of the patent, whichever period is longer. Applicants' assignee acknowledges its duty to replace the deposit should the depository be unable to furnish a sample when requested due to the condition of the deposit.

Other embodiments are within the following claims.

## Claims

1. A method for producing a cholera vaccine comprising growing a culture of V. cholerae cells having a mutated htx gene in growth medium wherein the toxin-coregulated pilus is present at a concentration representing 1% or more of total cell protein and inoculating an animal with cells or cell components from said culture.

2. A method according to claim 1 wherein the cells are grown in a growth medium having a pH of about 6.5 or less.

3. A method for producing a cholera vaccine comprising growing a culture of V. cholerae cells in growth medium at a pH of about 6.5 or less wherein the toxin-coregulated pilus is present at a concentration representing 1% or more of total cell protein and inoculating an animal with cells or cell components from said culture.

4. A method for producing a cholera vaccine according to any of claims 1 to 3 further comprising growing said culture of V. cholerae cells to stationary phase in growth medium that contains one or more of the amino acids: asparagine, arginine, serine, glutamic acid, or glutamine, said medium having an ionic strength equivalent to 50-100 mM NaC1, and wherein said medium is maintained at a temperature of 22-30°C, with exposure to oxygen.

5. A method for producing a cholera vaccine, according to any of claims 1 to 4, further comprising killing said cells, and providing said killed cells in a pharmaceutically acceptable vehicle.

6. A method for producing a cholera vaccine according to claim any of claims 1 to 5, said vaccine comprising toxin-coregulated pilus, wherein said method further comprises harvesting said V. cholerae and purifying said toxin-coregulated pilus from said cells.

7. A method for producing a cholera vaccine, according to any of claims 1 to 6, wherein said vaccine comprises a B subunit of cholera toxin and said method further comprises harvesting V. cholerae cells and purifying said B subunit from said cells.

8. A method according to any of claims 1 to 7, wherein said cells have non-toxic levels of cholera toxin.

9. A method according to any of claims 1, 2 or 4 to 8, wherein said culture comprises the strain deposited in the ATCC and assigned the number 53613.

10. A method according to any of claims 3 to 8, wherein said culture comprises the strain deposited in the ATCC and assigned the number 67396.

11. A solution comprising purified whole bacterial cells having at least 1% of toxin-coregulated pilus per cell as total protein.

12. A bacterial cell comprising at least 1% total protein as toxin-coregulated pilus, said cell lacking toxic levels of cholera toxin.

13. A cell according to claim 12, wherein said cell is a vibrio cholerae.

14. A cell according to claim 12 or claim 13, wherein said cell has a mutated htx gene.

## Patentansprüche

1. Verfahren zum Herstellen eines Choleravaccins, wobei es zu dem Verfahren gehört, dass man eine Kultur von V. Cholerazellen mit einem mutierten htx-Gen in einem Kulturmedium wachsen lässt, in dem die Toxin-koregulierten Pili in einer Konzentration entsprechend 1% oder mehr bezogen auf das gesamte Zellprotein vorhanden ist und dass ein Tier mit den Zellen oder Zellkomponenten aus dieser Kultur geimpft wird.

2. Verfahren nach Anspruch 1, bei dem die Zellen in einem Kulturmedium wachsen gelassen werden, das einen pH-Wert von etwa 6,5 oder niedriger aufweist.

3. Verfahren zum Herstellen eines Choleravaccins, wobei es zu dem Verfahren gehört, dass man eine Kultur von V. Cholerazellen bei einem pH-Wert von etwa 6,5 oder niedriger in einem Kulturmedium wachsen lässt, in dem die Toxin-koregulierten Pili in einer Konzentration entsprechend 1% oder mehr bezogen auf das gesamte Zellprotein vorhanden ist und dass ein Tier mit den Zellen oder Zellkomponenten aus der Kultur geimpft wird.

4. Verfahren zum Herstellen eines Choleravaccins nach einem der Ansprüche 1 bis 3, zu dem es ferner gehört, dass man die Kultur der V. Cholerazellen bis zur stationären Phase in dem Kulturmedium wachsen lässt, das eine oder mehrere der Aminosäuren, wie Asparagin, Arginin, Serin, Glutaminsäure oder Glutamin enthält, wobei das Medium eine Ionenstärke entsprechend 50-100 mM NaCl aufweist und auf einer Temperatur zwischen 22-30°C gehalten sowie Sauerstoff ausgesetzt ist.

5. Verfahren zum Herstellen eines Choleravaccins nach einem der Ansprüche 1 bis 4, wobei es zu dem Verfahren ferner gehört, dass die Zellen abgetötet und die abgetöteten Zellen in einem pharmazeutisch verträglichen Vehikel bereitgestellt werden.

6. Verfahren zum Herstellen eines Choleravaccins nach einem der Ansprüche 1 bis 5, wobei das Vaccin Toxin-koregulierte Pili enthält und es ferner zu dem Verfahren gehört, dass die V. Cholerazellen geerntet und die Toxin-koregulierten Pili von den Zellen gereinigt werden.

7. Verfahren zum Herstellen eines Choleravaccins nach einem der Ansprüche 1 bis 6, wobei das Vaccin die B-Untereinheiten des Choleratoxins enthält und es zu dem Verfahren ferner gehört, dass die V. Cholerazellen geerntet werden und die B-Untereinheiten von den Zellen gereinigt wird.

8. Verfahren nach einem der Ansprüche 1 bis 7, bei dem die Zellen nicht-toxische Werte des Choleratoxins enthalten.

9. Verfahren nach einem der Ansprüche 1, 2 oder 4 bis 8, bei dem die Kultur den Stamm enthälten, der bei der ATCC hinterlegt ist und die Nummer 53613 trägt.

10. Verfahren nach einem der Ansprüche 3 bis 8, bei dem die Kultur den Stamm enthält, der bei der ATCC hinterlegt ist und die Nummer 67396 trägt.

11. Lösung, die gereinigte bakterielle Vollzellen mit wenigstens 1% Toxin-koregulierten Pili pro Zelle als Gesamtprotein enthält.

12. Bakterienzelle, bei der wenigstens 1% des gesamten Proteins als Toxin-koregulierter Pili vorliegt und der Zelle toxische Werte von Choleratoxin fehlen.

13. Zelle nach Anspruch 12, bei der die Zelle eine Vibriocholerazelle ist.

14. Zelle nach Anspruch 12 oder 13, bei der die Zelle ein mutiertes htx-Gen enthält.

## Revendications

1. Procédé de production d'un vaccin cholérique comprenant le développement d'une culture cellulaire de V.Cholerae ayant un gène htx muté dans un milieu de culture dans lequel le pilus corégulé par des toxines est présent à une concentration représentant 1 % ou plus de la teneur protéique cellulaire totale et l'inoculation d'un animal avec les cellules ou les composants cellulaires à partir de ladite culture.

2. Procédé selon la revendication 1, dans lequel les cellules sont cultivées dans un milieu de culture ayant un pH d'environ 6,5 ou inférieur.

3. Procédé de production d'un vaccin cholérique comprenant le développement d'une culture cellulaire de V.Cholerae dans un milieu de culture à un pH d'environ 6,5 ou inférieur dans lequel le pilus corégulé par des toxines est présent à une concentration représentant 1 % ou plus, de la teneur protéique cellulaire totale et l'inoculation d'un animal avec les cellules ou les composants cellulaires à partir de ladite culture.

4. Procédé de production d'un vaccin cholérique selon l'une quelconque des revendications 1 à 3, comprenant en outre le développement de ladite culture de V.Cholerae jusqu'à la phase stationnaire dans un milieu de culture qui contient un ou plusieurs des acides aminés : asparagine. arginine, sérine, acide glutamique, ou glutamine, ledit milieu ayant une force ionique équivalente à 50-100 mM NaCI et étant maintenu à une température de 22-30 °C sous exposition d'oxygène.

5. Procédé de production d'un vaccin cholérique selon l'une quelconque des revendications 1 à 4, comprenant en outre la destruction desdites cellules et la fourniture desdites cellules tuées dans un véhicule pharmaceutiquement acceptable.

6. Procédé de production d'un vaccin cholérique selon l'une quelconque des revendications 1 à 5, ledit vaccin comprenant un pilus corégulé par des toxines, ledit procédé comprenant en outre la récupération desdites cellules de V.Cholerae et la purification dudit pilus corégulé par des toxines à partir desdites cellules.

7. Procédé de production d'un vaccin cholérique selon l'une quelconque des revendications 1 à 6, dans lequel ledit vaccin comprend une sous-unité B de toxine cholérique, ledit procédé comprenant en outre la récupération des cellules de V.Cholerae et la purification de ladite sous-unité B à partir desdites cellules.

8. Procédé selon l'une quelconque des revendications 1 à 7, dans lequel lesdites cellules possèdent des niveaux non-toxiques de toxine cholérique.

9. Procédé selon l'une quelconque des revendications 1,2 ou 4 à 8, dans lequel ladite culture comprend la souche déposée à l'ATCC sous le numéro 53613.

10. Procédé selon l'une quelconque des revendications 3 à 8, dans lequel ladite culture comprend la souche déposée à l'ATCC sous le numéro 67396.

11. Solution comprenant des cellules bactériennes entières purifiées ayant au moins 1 % de pilus corégulé par des toxines par cellule par rapport aux protéines totales.

12. Cellule bactérienne comprenant au moins 1 % de pilus corégulé par des toxines par rapport aux protéines totales, ladite cellule étant dépourvue de niveaux toxiques de toxine cholérique.

13. Cellule selon la revendication 12, ladite cellule étant un vibrio cholerae.

14. Cellule selon la revendication 12 ou la revendication 13, ladite cellule ayant un gène htx muté.
